# FASCICULE DE BREVET EUROPEEN

(11) **EP 0 966 946 B1**
(45) Date de publication et mention de la délivrance du brevet: **10.11.2004**
(21) Numéro de dépôt: 99401371.2
(22) Date de dépôt: 08.06.1999
(51) Int. Cl.: A61K 7/06

(54) **Composition cosmétique comprenant au moins un polyester sulfonique ramifié et au moins un agent de conditionnement**
Kosmetische Zusammensetzung, die mindestens einen verzweigten Sulfopolyester und mindestens ein Konditioniermittel enthält
Cosmetic composition at least containing a branched sulfonic polyester and a conditioning agent

(30) Priorité: 11.06.1998 FR 9807375
(43) Date de publication de la demande: 29.12.1999
(73) Titulaire: L'OREAL, 75008 Paris (FR)
(72) Inventeur: Rollat-Corvol, Isabelle, 92100 Boulogne-Billancourt (FR); Samain, Henri, 91570 Bievres (FR)
(74) Mandataire: Miszputen, Laurent

(56) Documents cités:
- EP-A- 0 551 749
- EP-A- 0 679 386
- EP-A- 0 792 636
- EP-A- 0 864 319
- WO-A-99/63955
- DE-A- 3 411 062
- FR-A- 2 745 175
- FR-A- 2 760 636
- US-A- 4 335 220
- US-A- 5 320 836
- BASE DE DONN ES "CHEMICAL ABSTRACTS" (SERVEUR: STN, Karlsruhe, DE); abrégé 115: 78 621; & JP 02 218 797 A (KAO CORP.)31 AOUT 1990 XP002116298
- DATABASE CTFA ONLINE [en ligne] 'POLYCARE PS20'

## Description

L'invention a pour objet une composition cosmétique pour les fibres kératiniques telles que les cheveux, comprenant au moins un polyester sulfonique ramifié particulier et au moins un agent de conditionnement choisi dans le groupe comprenant les silicones non volatiles, les polymères cationiques ou amphotères et les tensioactifs cationiques ou amphiphiles. Elle vise également un procédé de traitement des fibres kératiniques telles que les cheveux, en particulier un procédé de fixation et/ou de maintien de la coiffure mettant en oeuvre ladite composition ainsi que l'utilisation de cette composition dans ou pour la fabrication d'une formulation cosmétique de coiffage.

Par "fibres kératiniques", on entend au sens de la présente invention, les cheveux, les cils et les sourcils.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

On connaît également les gels ou les mousses de coiffage qui sont généralement appliqués sur les cheveux mouillés avant de faire un brushing ou une mise en plis. A la différence des laques aérosols classiques, ces compositions présentent l'inconvénient de ne pas permettre la fixation des cheveux dans une forme déjà réalisée. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage.

La plupart des compositions de l'état de la technique présentent le même inconvénient de ne pas permettre facilement à l'utilisateur de recoiffer les cheveux en leur redonnant leur forme initiale lorsque la coiffure a été défaite sous l'effet d'une contrainte. Dans ce cas, il est souvent nécessaire de recommencer l'ensemble des opérations de coiffage et de fixation si l'on souhaite retrouver la coiffure initiale.

On recherche donc des compositions de coiffage qui permettent de remettre facilement en forme la coiffure lorsque celle-ci a été défaite, par exemple par un coup de vent ou par une forte agitation. On parle de pouvoir "recoiffant", c'est-à-dire de la possibilité de remettre facilement en forme une coiffure avec le moins de geste possible, sans l'aide d'un peigne ou d'une brosse lorsque cette coiffure a été défaite.

Un autre inconvénient des compositions de l'état de la technique est que celles-ci ne permettent pas toujours de discipliner convenablement tous les cheveux, c'est-à-dire de leur faire prendre à tous la même forme et la même direction. Il en résulte parfois une impression de désordre de la coiffure et d'aspect indiscipliné. On recherche donc des compositions procurant un effet de discipline suffisamment fort pour que la chevelure ait un aspect soigné.

Enfin, les compositions destinées à la fixation de la coiffure présentent parfois l'inconvénient d'altérer les propriétés cosmétiques des cheveux. Ainsi, les cheveux peuvent devenir rêches, difficiles à démêler, perdre leur toucher et leur aspect agréables ou encore manquer de corps. On recherche donc des compositions coiffantes procurant de bonnes propriétés cosmétiques, notamment en terme de démêlage, de douceur, de toucher et de corps. Au sens de la présente invention, on dira que la chevelure a du "corps" si elle procure au toucher à la main une sensation d'effet de masse et de légère rigidité.

Le document EP 551 749 décrit une composition capillaire contenant une résine polymérique dispersible et insoluble dans l'eau avec un polymère amphotère soluble dans l'eau. La résine polymérique peut être un polyester fonctionalisé avec des groupes sulphoniques, comme certaines résines AQ de la Société Kodak. Ce document antérieur ne mentionne que des polyesters sulfoniques linéaires.

Le document EP 609 386 décrit une composition de coiffage contenant une résine polymérique dispersible de type polyester, une huile hydrocarbonée et un dérivé silicone. Ce document antérieur ne mentionne, lui aussi, que des polyesters sulfoniques linéaires.

Il existe donc un besoin de trouver des compositions cosmétiques, notamment pour le coiffage, qui ne présentent pas l'ensemble des inconvénients indiqués ci-dessus.

De manière surprenante et inattendue, la Demanderesse a découvert que lorsque l'on associe des polyesters sulfoniques ramifiés avec des agents de conditionnement, il est possible d'obtenir des compositions cosmétiques répondant aux exigences exprimées ci-dessus.

L'invention a donc pour objet une composition cosmétique comprenant, dans un milieu cosmétiquement acceptable, au moins un polyester sulfonique ramifié et au moins un agent de conditionnement choisi dans le groupe comprenant les silicones non volatiles, les polymères cationiques ou amphotères et les tensioactifs cationiques ou amphiphiles.

Les polyesters sulfoniques ramifiés particulièrement visés par la présente invention sont ceux décrits dans les demandes de brevets WO 95/18191, WO97/08261 et WO 97/20899.

Les polyesters sulfoniques ramifiés sont formés par polymérisation de:
(i) au moins un acide dicarboxylique difonctionnel ne portant pas de fonction sulfonique;
(ii) au moins un monomère difonctionnel portant au moins une fonction sulfonique, le ou les groupements fonctionnels étant choisis dans le groupe comprenant les groupements hydroxyle, carboxyle et amino;
(iii) au moins un diol ou un mélange de diol (s) et de diamine (s);
(iv) éventuellement un monomère difonctionnel choisi dans le groupe comprenant les hydroxyacides carboxyliques, les aminoacides carboxyliques et leurs mélanges;
(v) au moins un réactif multifonctionnel portant au moins trois groupements fonctionnels choisis dans le groupe comprenant les groupements amino, alcool et acide carboxylique.

Cette polymérisation est avantageusement effectuée à partir de:
(i) au moins un acide dicarboxylique difonctionnel ne portant pas de fonction sulfonique;
(ii) 2 à 15 % relatif en mole de monomère difonctionnel portant au moins une fonction sulfonique;
(iii) au moins un diol ou un mélange de diol (s) et de diamine (s);
(iv) 0 à 40 % relatif en mole du monomère difonctionnel choisi dans le groupe comprenant les hydroxyacides carboxyliques, les aminoacides carboxyliques et leurs mélanges;
(v) 0,1 à 40 % relatif en mole du réactif multifonctionnel portant au moins trois groupements fonctionnels réactifs.

Les polymères sulfoniques ramifiés contiennent avantageusement des proportions substantiellement égales, en nombre d'équivalents, d'une part en fonctions acide carboxylique et, d'autre part en fonctions diol et/ou diol et diamine.

De préférence, on choisit, comme polymères sulfoniques ramifiés, les polymères AQ 1350, AQ 1045, AQ 1950 ou AQ 14 000 commercialisés par la Société Eastman, et de préférence le polymère AQ 1350.

L'acide dicarboxylique difonctionnel (i) pourra être avantageusement choisi dans le groupe comprenant les acides aliphatiques dicarboxyliques, les acides alicycliques dicarboxyliques, des acides aromatiques dicarboxyliques et un mélange de ceux-ci. Plus particulièrement, l'acide dicarboxylique (i) pourra être choisi dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique.

Le monomère difonctionnel (ii) tel que défini ci-dessus est avantageusement choisi dans le groupe comprenant les acides dicarboxylique, les esters d'acide dicarboxylique, les glycols et les hydroxyacides contenant, chacun, au moins un groupement métal sulfonate.

Le diol (iii) est de préférence choisi dans le groupe comprenant les alcanediols et les polyalkylènediols et plus particulièrement, ce diol (iii) est choisi dans le groupe comprenant l'éthylène glycol, le propylène glycol, le diéthylène glycol, le triéthylène glycol et le polyproprylène glycol.

La diamine (iii) peut avantageusement être choisie dans le groupe comprenant les alcanediamines et lespolyalkylènediamines et le réactif multifonctionnel (v) est de préférence choisi dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

La composition comprend avantageusement, en pourcentage relatif en poids de la composition, de 0,01 à 40 % de polyester sulfonique ramifié et de préférence, de 0,1 à 16 % de polyester sulfonique ramifié, et plus préférentiellement de 0,5 à 8 % de polyester sulfonique ramifié.

La composition comprend avantageusement, en pourcentage relatif en poids de la composition, de 0,01 à 16 % d'agent de conditionnement et de préférence, de 0,05 à 4 %.

Le ou les polyesters sulfoniques ramifiés peuvent se présenter sous forme solubilisée ou sous forme de dispersion de particules solides.

On peut choisir avantageusement un agent de conditionnement insoluble dans le groupe comprenant les poly-α-oléfines, les huiles fluorées, les huiles végétales, les cires naturelles, les cires fluorées, les gommes fluorées et les esters d'acides gras, les silicones insolubles, les composés amides comportant au moins une chaîne grasse; lesdits agents pouvant être présents sous forme de mélanges.

Lorsque l'agent de conditionnement est un polymère cationique ou amphotère, il est avantageusement choisi dans le groupe formé par :
(a) les dérivés cationiques cellulosiques ;
(a) les homopolymères et les copolymères d'halogénure de diméthyldiallylammonium;
(c) les homopolymères et copolymères d'halogénure de méthacryloyloxy et éthyltriméthylammonium ;
(d) les polymères polyammonium quaternaire ;
(e) les copolymères de vinylpyrrolidone à motifs cationi ques;
(f) les polysiloxanes cationiques.

Le polymère cationique est avantageusement choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quaternisés ou non, les polymères quaternaires de vinylpyrrolidone et de vinylimidazole, les polyamidoamines et leurs mélanges.

On peut choisir avantageusement des agents tensioactifs cationiques insolubles dans l'eau dans le groupe constitué par les amines grasses et leurs sels ainsi que les sels d'ammonium quaternaire. De préférence, les amines grasses sont choisis dans le groupe comprenant la dioctylamine, la stéaryldiméthylamine, la palmityl-diméthylamine, l'oléocétyldiméthylamine et les amidoamines telles que la stéarylamido-éthyldiéthylamine, la béhénylamidopropyldiméthylamine, la stérarylamidopropyldiméthyl-amine, l'oléylamido-propyldiméthylamine, la stéarylamidoéthyldiméthylarnine.

L'agent tensioactif cationique de type sel d'ammonium quaternaire est choisi avantageusement parmi ceux qui présentent la formule générale (I) suivante : 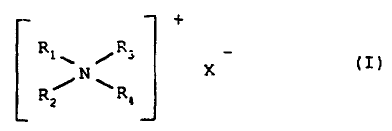 dans laquelle R₁ à R₄, identiques ou différents, représentent un radical aliphatique comportant de 1 à 22 atomes de carbone, ou un radical aromatique, alcoxy, polyoxyalkylène, alkylamide, hydroxyalkyle, aryle ou alkylaryle comportant de 12 à 22 atomes de carbone; X est un anion choisi dans le groupe des halogénures, phosphates, acétates, lactates ou alkylsulfates,
ou les sels d'ammonium quaternaire de l'imidazolinium, de formule (II) suivante : 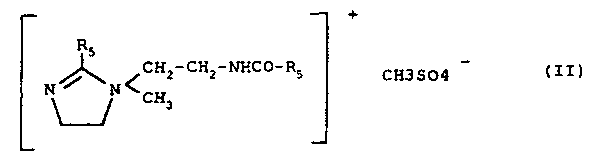 dans laquelle R₅ représente un mélange de radicaux alcényle et/ou alkyle comportant de 13 à 21 atomes de carbone et dérivés des acides gras du suif,
ou les sels de diammonium quaternaire de formule (III): 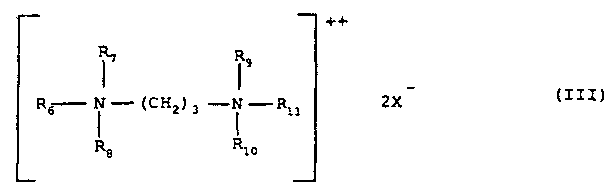 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.

La composition comprend avantageusement, en outre, des additifs cosmétiques usuels tels que des plastifiants ou des neutralisants et elle se présente sous forme de composition vaporisable, de mousse, de gel ou de lotion.

La composition comprend de préférence, en plus, un solvant choisi parmi l'eau, un alcool ou un mélange hydroalcoolique ainsi qu'une quantité appropriée de propulseur constitué avantageusement par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

Un autre objet de l'invention est un dispositif aérosol constitué par un récipient contenant une composition aérosol constituée par d'une part une phase liquide (ou jus) contenant une composition conforme à l'invention, dans un solvant approprié et un propulseur, ainsi qu'un moyen de distribution de ladite composition aérosol.

Encore un objet de l'invention est un procédé de traitement des fibres kératiniques, en particulier des cheveux, comprenant l'application sur lesdites fibres de la composition conforme à l'invention, avant ou après la mise en forme de la coiffure.

Encore un autre objet de l'invention concerne l'utilisation d'une composition définie ci-dessus dans ou pour la fabrication d'une formulation cosmétique de coiffage.

Les exemples ci-après illustrent la présente invention sans en limiter la portée.

On met en oeuvre les polymères indiqués ci-après.

| | |
|---|---|
| AQ 1350, AQ 1045, AQ1950 et AQ 14000 | Polyesters sulfoniques branchés commercialisés par Eastman |
| | |
| DC 190 | Polydiméthyl méthylsiloxane oxyéthyléné et oxypropyléné commercialisé par Dow Corning |
| | |
| DC 939 | Silicone cationique commercialisée par Dow Corning |

### Exemple 1:

On compare les performances obtenues avec des compositions conformes à l'invention et avec une compositions conforme à l'art antérieur ne contenant pas d'agent de conditionnement. Les performances comparées sont le démêlage, le corps, la douceur, le toucher, le coiffant et le recoiffant.

| **Composition 1 (invention):** | |
|---|---|
| AQ 1350 | 4g |
| DOW CORNING 190 SURFACTANT | 0,4 g 5Matière active) |
| Eau qs | 100 g |

| **Composition 2 (invention):** | |
|---|---|
| AQ 1350 | 4g |
| DC 939 | 0,4 g |
| Eau qs | 100 g |

| **Composition 3 (art antérieur)** | |
|---|---|
| AQ 1350 | 4g |
| Eau qs | 100 g |

On dispose pour cet essai de tête malléables. Les compositions sont appliquée par demi tète, c'est-à-dire sur le côté droite ou gauche de la chevelure séparée par une raie au milieu.

On dépose 3 grammes de produit par demi-tête, dont les cheveux ont été préalablement shampooinés puis essorés. Les têtes malléables subissent un brushing, on les coiffe à 21 °C et 30 % d'humidité relative et on les laisse 10 minutes au repos dans ces mêmes conditions de température et d'humidité.

On mesure les performances obtenues au moyen d'un test sensoriel. Les performances sont notées entre 0 (mauvais) et 5 (excellent). Elles sont rassemblées dans le tableau 1 ci-après.

**Tableau 1**

| Comparatifs ⇒ | Composition1 / Composition 3 | Composition2 / Composition 3 |
|---|---|---|
| DEMELAGE | 4.5 / 3.5 | 5.0 / 2.0 |
| CORPS | 1.5 / 0.5 | 1.5 / 0.5 |
| DOUCEUR | 4.0 / 2.5 | 3.5 / 1.5 |
| TOUCHER | 4.0 / 2.0 | 4.0 / 1.0 |
| COIFFANT | 4.0 / 2.0 | 4.5 / 3.5 |
| RECOIFFANT | 4.0 / 0.0 | 4.0 / 1.0 |

On observe que les performances obtenues par les compositions 1 et 2 sont nettement meilleures que celles obtenues avec composition 3. En particulier, l'effet recoiffant est nettement amélioré ainsi que le corps de la coiffure.

### Exemple 2:

On réalise trois compositions conformes à l'invention.

| **Composition 5 (invention):** | |
|---|---|
| AQ 1045 | 1,2g |
| DOW CORNING 190 SURFACTANT | 0,2 g (Matière active) |
| Eau qs | 100 g |

| **Composition 6 (invention):** | |
|---|---|
| AQ 1950 | 8,3g |
| DC 939 | 1 g |
| Eau qs | 100 g |

| **Composition 7 (invention):** | |
|---|---|
| AQ 14000 | 10g |
| DC 939 | 1 g |
| Eau qs | 100 g |

On applique les trois compositions 5, 6 et 7 sur des cheveux sensibilisées (décolorées), on laisse sécher et on brosse les cheveux.

On observe qu'elles procurent un effet adoucissant important et que les cheveux sont particulièrement brillants et faciles à démêler.

## Revendications

1. Composition cosmétique pour les fibres kératiniques telles que les cheveux, **caractérisée par le fait qu'**elle comprend, dans un milieu cosmétiquement acceptable, au moins un polyester sulfonique ramifié et au moins un agent de conditionnement choisi dans le groupe comprenant les silicones non volatiles, les polymères cationiques ou amphotères et les tensioactifs cationiques ou amphiphiles, le polyester sulfonique ramifié est formé par polymérisation de:
(i) au moins un acide dicarboxylique difonctionnel ne portant pas de fonction sulfonique;
(ii) au moins un monomère difonctionnel portant au moins une fonction sulfonique, le ou les groupements fonctionnels étant choisis dans le groupe comprenant les groupements hydroxyle, carboxyle et amino;
(iii) au moins un diol ou un mélange de diol (s) et de diamine (s);
(iv) éventuellement un monomère difonctionnel choisi dans le groupe comprenant les hydroxyacides carboxyliques, les aminoacides carboxyliques et leurs mélanges;
(v) au moins un réactif multifonctionnel portant au moins trois groupements fonctionnels choisis dans le groupe comprenant les groupements amino, alcool et acide carboxylique.

2. Composition selon la revendication 1, **caractérisée par le fait que** la polymérisation est effectué à partir de:
(i) au moins un acide dicarboxylique difonctionnel ne portant pas de fonction sulfonique;
(ii) 2 à 15 % relatif en mole de monomère difonctionnel portant au moins une fonction sulfonique;
(iii) au moins un diol ou un mélange de diol (s) et de diamine (s);
(iv) 0 à 40 % relatif en mole du monomère difonctionnel choisi dans le groupe comprenant les hydroxyacides carboxyliques, les aminoacides carboxyliques et leurs mélanges;
(v) 0,1 à 40 % relatif en mole du réactif multifonctionnel portant au moins trois groupements fonctionnels réactifs.

3. Composition selon l'une quelconque des revendications 1 ou 2, **caractérisée par le fait que** le polymère contient des proportions substantiellement égales, en nombre d'équivalents, d'une part en fonctions acide carboxylique et, d'autre part en fonctions diol et/ou diol et diamine.

4. Composition selon l'une quelconque des revendications 1 à 3, **caractérisée par le fait que** l'acide dicarboxylique difonctionnel ne portant pas de fonction sulfonique (i) est choisi dans le groupe comprenant les acides aliphatiques dicarboxyliques, les acides alicycliques dicarboxyliques, des acides aromatiques dicarboxyliques et un mélange de ceux-ci.

5. Composition selon la revendication 4, **caractérisée par le fait que** l'acide dicarboxylique difonctionnel ne portant pas de fonction sulfonique (i) est choisi dans le groupe comprenant l'acide 1,4 - cyclohexanedioïque, l'acide succinique, l'acide glutarique, l'acide adipique, l'acide azelaïque, acide sebacique, l'acide fumarique, l'acide maléïque, l'acide 1,3 - cyclohexanedioïque, l'acide phtalique, l'acide téréphtaltique et l'acide isophtalique et leurs mélanges.

6. Composition selon l'une quelconque des revendications 1 à 3, **caractérisée par le fait que** le monomère difonctionnel (ii) portant au moins une fonction sulfonique est choisi dans le groupe comprenant les acides dicarboxyliques, les esters d'acide dicarboxylique, les glycols et les hydroxyacides contenant, chacun, au moins un groupement métal sulfonate.

7. Composition selon l'une quelconque des revendications 1 à 3, **caractérisée par le fait que** le diol (iii) est choisi dans le groupe comprenant les alcanediols et les polyalkylènediols.

8. Composition selon la revendication 7, **caractérisée par le fait que** le diol (iii) est choisi dans le groupe comprenant l'éthylène glycol, le propylène glycol, le diéthylène glycol, le triéthylène glycol et le polyproprylène glycol.

9. Composition selon l'une quelconque des revendications 1 à 3, **caractérisée par le fait que** la diamine (iii) est choisie dans le groupe comprenant les alcanediamines et les polyalkylènediamines.

10. Composition selon l'une quelconque des revendications 1 à 3, **caractérisée par le fait que** le réactif multifonctionnel (v) est choisi dans le groupe comprenant le triméthyloléthane, le triméthylolpropane, le glycérol, le pentaérythritol, le sorbitol, l'anhydride trimellitique, l'érythritol, le threitol, le dipentaérythritol, le dianhydride pyromellitique et l'acide diméthylpropionique.

11. Composition selon l'une quelconque des revendications précédentes, **caractérisée par le fait qu'**elle comprend, en pourcentage relatif en poids de la composition, de 0,01 à 40 % de polyester sulfonique ramifié et de préférence, de 0,1 à 16 % de polyester sulfonique ramifié, et plus préférentiellement encore de 0,5 à 8% de polyester sulfonique ramifié.

12. Composition selon l'une quelconque des revendications précédentes, **caractérisée par le fait qu'**elle comprend, en pourcentage relatif en poids de la composition, de 0,01 à 16 % d'agent de conditionnement et de préférence, de 0,05 à 4 %.

13. Composition selon l'une quelconque des revendications précédentes, **caractérisée par le fait que** le ou les polyesters sulfoniques ramifiés se présentent sous forme solubilisée ou sous forme de dispersion de particules solides.

14. Composition selon l'une quelconque des revendications précédentes, **caractérisée par le fait que** l'agent de conditionnement est insoluble et est choisi dans le groupe comprenant les poly-α-oléfines, les huiles fluorées, les huiles végétales, les cires naturelles, les cires fluorées, les gommes fluorées et les esters d'acides gras, les silicones insolubles, les composés amides comportant au moins une chaîne grasse; lesdits agents pouvant être présents sous forme de mélanges.

15. Composition selon la revendication 1, **caractérisée par le fait que** le polymère cationique ou amphotère choisi dans le groupe formé par :
(a) les dérivés cationiques cellulosiques ;
(b) les homopolymères et les copolymères d'halogénure de diméthyldiallylammonium;
(c) les homopolymères et copolymères d'halogénure de méthacryloyloxy - éthyltriméthylammonium ;
(d) les polymères polyammonium quaternaire ;
(e) les copolymères de vinylpyrrolidone à motifs cationiques
(vi) les polysiloxanes cationiques.

16. Composition selon la revendication 15, **caractérisée par le fait que** le polymère cationique est choisi parmi les dérivés d'éther de cellulose quaternaires; les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quaternisés ou non, les polymères quaternaires de vinylpyrrolidone et de vinylimidazole, les polyamidoamines et leurs mélanges.

17. Composition selon l'une quelconque des revendications précédentes, **caractérisé par le fait que** les agents tensioactifs cationiques sont insolubles dans l'eau et sont choisis dans le groupe constitué par les amines grasses et leurs sels ainsi que les sels d'ammonium quaternaire.

18. Composition selon la revendication 17, **caractérisée par le fait que** les amines grasses sont choisis dans le groupe comprenant la dioctylamine, la stéaryldiméthylamine, la palmityl-diméthylamine, l'oléocétyldiméthylamine et les amidoamines telles que la stéarylamido-éthyldiéthylamine, la béhénylamidopropyldiméthylamine, la stéarylamidopropyldiméthyl-amine, l'oléylamidopropyl - diméthylamine, la stéarylamidoéthyldiméthylamine.

19. Composition selon la revendication 17, **caractérisée par le fait que** ledit agent tensioactif cationique de type sel d'ammonium quaternaire est choisi parmi ceux qui présentent la formule générale (I) suivante : 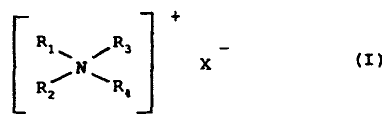 dans laquelle R₁ à R₄, identiques ou différents, représentent un radical aliphatique comportant de 1 à 22 atomes de carbone, ou un radical aromatique, alcoxy, polyoxyalkylène, alkylamide, hydroxyalkyle, aryle ou alkylaryle comportant de 12 à 22 atomes de carbone; X est un anion choisi dans le groupe des halogénures, phosphates, acétates, lactates ou alkylsulfates,
ou les sels d'ammonium quaternaire de l'imidazolinium, de formule (II) suivante : 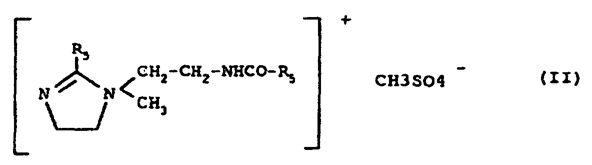 dans laquelle R₅ représente un mélange de radicaux alcényle et/ou alkyle comportant de 13 à 21 atomes de carbone et dérivés des acides gras du suif,
ou les sels de diammonium quaternaire de formule (III): 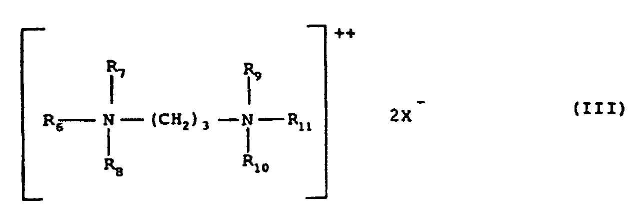 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.

20. Composition selon l'une quelconque des revendications précédentes, **caractérisée par le fait qu'**elle comprend, en outre, des additifs cosmétiques usuels tels que des plastifiants ou des neutralisants.

21. Composition selon l'une quelconque des revendications précédentes, **caractérisée par le fait qu'**elle se présente sous forme de composition vaporisable, de mousse, de gel ou de lotion.

22. Composition selon l'une quelconque des revendications précédentes, **caractérisée par le fait qu'**elle comprend un solvant choisi parmi l'eau, un alcool ou un mélange hydroalcoolique.

23. Composition selon l'une quelconque des revendications précédentes, **caractérisée en ce qu'**elle comprend en outre une quantité appropriée de propulseur.

24. Composition selon la revendication 23, **caractérisée en ce que** le propulseur est constitué par les gaz comprimés ou liquéfiés usuels, de préférence l'air, le gaz carbonique ou l'azote comprimés, ou encore un gaz soluble ou non dans la composition tel que le diméthyl éther, les hydrocarbures fluorés ou non, et leurs mélanges.

25. Dispositif aérosol constitué par un récipient contenant une composition aérosol constituée par d'une part une phase liquide (ou jus) contenant une composition selon l'une quelconque des revendications 1 à 20 dans un solvant approprié et un propulseur, ainsi qu'un moyen de distribution de ladite composition aérosol.

26. Procédé de traitement des fibres kératiniques, en particulier des cheveux, **caractérisé en ce qu'**on applique sur lesdites fibres la composition telle que définie dans les revendications 1 à 24, avant ou après la mise en forme de la coiffure.

27. Utilisation d'une composition selon l'une quelconque des revendications 1 à 24 dans ou pour la fabrication d'une formulation cosmétique de coiffage.

## Claims

1. Cosmetic composition for keratin fibres such as the hair, **characterized in that** it comprises, in a cosmetically acceptable medium, at least one branched sulphonic polyester and at least one conditioning agent chosen from the group comprising non-volatile silicones, cationic or amphoteric polymers and cationic or amphiphilic surfactants, the branched sulphonic polyester is formed by polymerization of:
(i) at least one difunctional dicarboxylic acid bearing no sulphonic functions;
(ii) at least one difunctional monomer bearing at least one sulphonic function, the functional group(s) being chosen from the group comprising hydroxyl, carboxyl and amino groups;
(iii) at least one diol or a mixture of diol(s) and of diamine(s);
(iv) optionally one difunctional monomer chosen from the group comprising hydroxycarboxylic acids, aminocarboxylic acids and mixtures thereof;
(v) at least one multifunctional reagent bearing at least three functional groups chosen from the group comprising amino, alcohol and carboxylic acid groups.

2. Composition according to Claim 1, **characterized in that** the polymerization is carried out using:
(i) at least one difunctional dicarboxylic acid bearing no sulphonic functions;
(ii) 2 to 15 mol% of difunctional monomer bearing at least one sulphonic function;
(iii) at least one diol or a mixture of diol(s) and of diamine(s);
(iv) 0 to 40 mol% of the difunctional monomer chosen from the group comprising hydroxycarboxylic acids, aminocarboxylic acids and mixtures thereof;
(v) 0.1 to 40 mol% of the multifunctional reagent bearing at least three reactive functional groups.

3. Composition according to either of Claims 1 and 2, **characterized in that** the polymer contains substantially equal proportions, in terms of number of equivalents, of carboxylic acid functions, on the one hand, and of diol and/or diol and diamine functions, on the other hand.

4. Composition according to any one of Claims 1 to 3, **characterized in that** the difunctional dicarboxylic acid bearing no sulphonic functions (i) is chosen from the group comprising aliphatic dicarboxylic acids, alicyclic dicarboxylic acids, aromatic dicarboxylic acids and a mixture thereof.

5. Composition according to Claim 4, **characterized in that** the difunctional dicarboxylic acid bearing no sulphonic functions (i) is chosen from the group comprising 1,4-cyclohexanedioic acid, succinic acid, glutaric acid, adipic acid, azelaic acid, sebacic acid, fumaric acid, maleic acid, 1,3-cyclohexanedioic acid, phthalic acid, terephthalic acid and isophthalic acid, and mixtures thereof.

6. Composition according to any one of Claims 1 to 3, **characterized in that** the difunctional monomer (ii) bearing at least one sulphonic function is chosen from the group comprising dicarboxylic acids, dicarboxylic acid esters, glycols and hydroxy acids each containing at least one metal sulphonate group.

7. Composition according to any one of Claims 1 to 3, **characterized in that** the diol (iii) is chosen from the group comprising alkanediols and polyalkylenediols.

8. Composition according to Claim 7, **characterized in that** the diol (iii) is chosen from the group comprising ethylene glycol, propylene glycol, diethylene glycol, triethylene glycol and polypropylene glycol.

9. Composition according to any one of Claims 1 to 3, **characterized in that** the diamine (iii) is chosen from the group comprising alkanediamines and polyalkylenediamines.

10. Composition according to any one of Claims 1 to 3, **characterized in that** the multifunctional reagent (v) is chosen from the group comprising trimethylolethane, trimethylolpropane, glycerol, pentaerythritol, sorbitol, trimellitic anhydride, erythritol, threitol, dipentaerythritol, pyromellitic dianhydride and dimethylpropionic acid.

11. Composition according to any one of the preceding claims, **characterized in that** it comprises, as a relative percentage by weight of the composition, from 0.01 to 40% of branched sulphonic polyester, preferably from 0.1 to 16% of branched sulphonic polyester, and even more preferably from 0.5 to 8% of branched sulphonic polyester.

12. Composition according to any one of the preceding claims, **characterized in that** it comprises, as a relative percentage by weight of the composition, from 0.01 to 16% of conditioning agent and preferably from 0.05 to 4%.

13. Composition according to any one of the preceding claims, **characterized in that** the branched sulphonic polyester(s) is (are) in dissolved form or in the form of a dispersion of solid particles.

14. Composition according to any one of the preceding claims, **characterized in that** the conditioning agent is insoluble and is chosen from the group comprising poly-α-olefins, fluoro oils, plant oils, natural waxes, fluoro waxes, fluoro gums, fatty acid esters, insoluble silicones and amide compounds comprising at least one fatty chain; it being possible for the said agents to be present in the form of mixtures.

15. Composition according to Claim 1, **characterized in that** the cationic or amphoteric polymer is chosen from the group formed by:
(a) cationic cellulose derivatives;
(b) dimethyldiallylammonium halide homopolymers and copolymers;
(c) methacryloyloxyethyltrimethylammonium halide homopolymers and copolymers;
(d) polyquaternary ammonium polymers;
(e) vinylpyrrolidone copolymers containing cationic units;
(f) cationic polysiloxanes.

16. Composition according to Claim 15, **characterized in that** the cationic polymer is chosen from quaternary cellulose ether derivatives, copolymers of cellulose with a water-soluble quaternary ammonium monomer, cyclopolymers, cationic polysaccharides, cationic silicone polymers, quaternized or non-quaternized vinylpyrrolidone/dialkylaminoalkyl acrylate or methacrylate copolymers, quaternary polymers of vinylpyrrolidone and of vinylimidazole, polyamidoamines and mixtures thereof.

17. Composition according to any one of the preceding claims, **characterized in that** the cationic surfactants are water-insoluble and are chosen from the group consisting of fatty amines and salts thereof, as well as quaternary ammonium salts.

18. Composition according to Claim 17, **characterized in that** the fatty amines are chosen from the group comprising dioctylamine, stearyldimethylamine, palmityldimethylamine, oleocetyldimethylamine and amidoamines such as stearylamidoethyldiethylamine, behenylamidopropyldimethylamine, stearylamidopropyldimethylamine, oleylamidopropyldimethylamine or stearylamidoethyldimethylamine.

19. Composition according to Claim 17, **characterized in that** the said cationic surfactant of quaternary ammonium salt type is chosen from those of general formula (I) below: 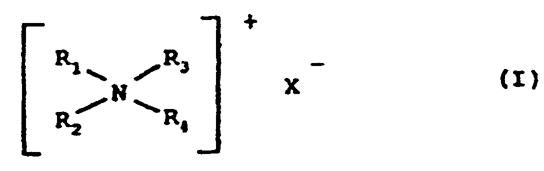 in which R₁ to R₄, which may be identical or different, represent an aliphatic radical comprising from 1 to 22 carbon atoms or an aromatic, alkoxy, polyoxyalkylene, alkylamide, hydroxyalkyl, aryl or alkylaryl radical comprising from 12 to 22 carbon atoms; X is an anion chosen from the group of halides, phosphates, acetates, lactates and alkylsulphates,
or the quaternary ammonium salts of imidazolinium, of formula (II) below: 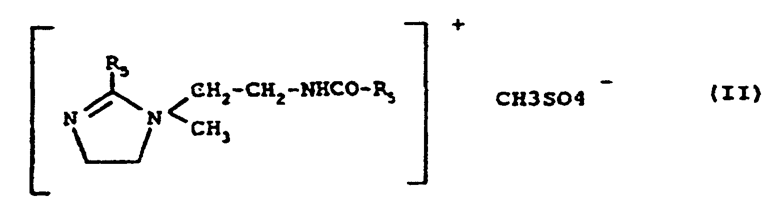 in which R₅ represents a mixture of alkenyl and/or alkyl radicals comprising from 13 to 21 carbon atoms and fatty acid derivatives of tallow,
or the quaternary diammonium salts of formula (III) : 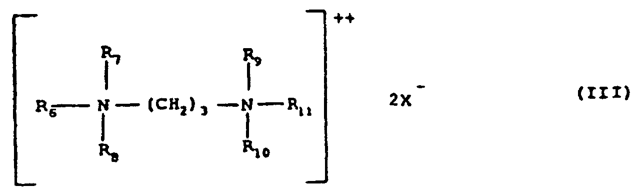 in which R₆ denotes an aliphatic radical comprising from 16 to 22 carbon atoms, R₇, R₈, R₉, R₁₀ and R₁₁ are chosen from hydrogen and an alkyl radical comprising from 1 to 4 carbon atoms, and X is an anion chosen from the group of halides, acetates, phosphates and sulphates.

20. Composition according to any one of the preceding claims, **characterized in that** it also comprises common cosmetic additives such as plasticizers or neutralizing agents.

21. Composition according to any one of the preceding claims, **characterized in that** it is in the form of a vaporizable composition, a mousse, a gel or a lotion.

22. Composition according to any one of the preceding claims, **characterized in that** it comprises a solvent chosen from water, an alcohol or an aqueous-alcoholic mixture.

23. Composition according to any one of the preceding claims, **characterized in that** it also comprises a suitable amount of propellant.

24. Composition according to Claim 23, **characterized in that** the propellant consists of the usual compressed or liquefied gases, preferably compressed air, carbon dioxide or nitrogen, or alternatively a gas which is soluble or insoluble in the composition, such as dimethyl ether, fluoro or non-fluoro hydrocarbons, and mixtures thereof.

25. Aerosol device consisting of a container containing an aerosol composition consisting, on the one hand, of a liquid phase (or fluid) containing a composition according to any one of Claims 1 to 20, in a suitable solvent and a propellant, as well as a means for dispensing the said aerosol composition.

26. Process for treating keratin fibres, in particular the hair, **characterized in that** the composition as defined in Claims 1 to 24 is applied to the said fibres, before or after shaping the hairstyle.

27. Use of a composition according to any one of Claims 1 to 24, in or for the manufacture of a cosmetic styling formulation.

## Patentansprüche

1. Kosmetische Zusammensetzung für Keratinfasern, wie die Haare, **dadurch gekennzeichnet, dass** sie in einem kosmetisch akzeptablen Medium mindestens einen verzweigten Sulfopolyester und mindestens ein Konditioniermittel enthält, das unter den nichtflüchtigen Siliconen, kationischen oder amphoteren Polymeren und kationischen oder amphiphilen grenzflächenaktiven Stoffen ausgewählt ist, wobei der verzweigte Sulfopolyester durch Polymerisation der folgenden Verbindungen gebildet wird:
(i) mindestens einer bifunktionellen Dicarbonsäure, die keine Sulfonsäurefunktion enthält;
(ii) mindestens eines bifunktionellen Monomers, das mindestens eine Sulfonsäurefunktion aufweist, wobei die funktionelle(n) Gruppe(n) unter den Hydroxygruppen, Carboxygruppen und Aminogruppen ausgewählt sind;
(iii) mindestens eines Diols oder eines Gemisches aus einem oder mehreren Diolen und einem oder mehreren Diaminen;
(iv) gegebenenfalls eines bifunktionellen Monomers, das unter den Hydroxycarbonsäuren, Aminocarbonsäuren und deren Gemischen ausgewählt ist; und
(v) mindestens eines multifunktionellen Reagens, das mindestens drei funktionelle Gruppen aufweist, die unter den Aminogruppen, Alkoholgruppen und Carbonsäuregruppen ausgewählt sind.

2. Zusammensetzung nach Anspruch 1, **dadurch gekennzeichnet, dass** die Polymerisation ausgehend von den folgenden Verbindungen durchgeführt wird:
(i) mindestens eine bifunktionelle Dicarbonsäure, die keine Sulfonsäurefunktion enthält;
(ii) 2 bis 15 Mol-% bifunktionelles Monomer mit mindestens einer Sulfonsäurefunktion;
(iii) mindestens ein Diol oder ein Gemisch aus einem oder mehreren Diolen und einem oder mehreren Diaminen;
(iv) 0 bis 40 Mol-% bifunktionelles Monomers, das unter den Hydroxycarbonsäuren, Aminocarbonsäuren und deren Gemischen ausgewählt ist; und
(v) 0,1 bis 40 Mol-% multifunktionelles Reagens mit mindestens drei reaktiven funktionellen Gruppen.

3. Zusammensetzung nach einem der Ansprüche 1 oder 2, **dadurch gekennzeichnet, dass** das Polymer bezogen auf die Anzahl der Äquivalente im Wesentlichen gleiche Mengenanteile an Carbonsäurefunktionen und Diolfunktionen und/oder Diol- und Diaminfunktionen enthält.

4. Zusammensetzung nach einem der Ansprüche 1 bis 3, **dadurch gekennzeichnet, dass** die bifunktionelle Dicarbonsäure (i), die keine Sulfonsäurefunktion enthält, unter den aliphatischen Dicarbonsäuren, alicyclischen Dicarbonsäuren, aromatischen Dicarbonsäuren und Gemischen dieser Säuren ausgewählt ist.

5. Zusammensetzung nach Anspruch 4, **dadurch gekennzeichnet, dass** die bifunktionelle Dicarbonsäure (i), die keine Sulfonsäurefunktion enthält, unter 1,4-Cyclohexandisäure, Bernsteinsäure, Glutarsäure, Adipinsäure, Azelainsäure, Sebacinsäure, Fumarsäure, Maleinsäure, 1,3-Cyclohexandisäure, Phthalsäure, Terephthalsäure und Isophthalsäure und deren Gemischen ausgewählt ist.

6. Zusammensetzung nach einem der Ansprüche 1 bis 3, **dadurch gekennzeichnet, dass** das bifunktionelle Monomer (ii), das mindestens eine Sulfonsäuregruppe aufweist, unter den Dicarbonsäuren, Dicarbonsäureestern, Glykolen und Hydroxysäuren ausgewählt ist, die jeweils mindestens eine Metallsulfonatgruppe enthalten.

7. Zusammensetzung nach einem der Ansprüche 1 bis 3, **dadurch gekennzeichnet, dass** das Diol (iii) unter den Alkandiolen und Polyalkylendiolen ausgewählt ist.

8. Zusammensetzung nach Anspruch 7, **dadurch gekennzeichnet, dass** das Diol (iii) unter Ethylenglykol, Propylenglykol, Diethylenglykol, Triethylenglykol und Polypropylenglykol ausgewählt ist.

9. Zusammensetzung nach einem der Ansprüche 1 bis 3, **dadurch gekennzeichnet, dass** das Diamin (iii) unter den Alkandiaminen und Polyalkylendiaminen ausgewählt ist.

10. Zusammensetzung nach einem der Ansprüche 1 bis 3, **dadurch gekennzeichnet, dass** das multifunktinelle Reagens (v) unter Trimethylolethan, Trimethylolpropan, Glycerin, Pentaerythrit, Sorbit, Trimellitsäureanhydrid, Erythrit, Threit, Dipentaerythrit, Pyromellitsäuredianhydrid und Dimethylpropionsäure ausgewählt ist.

11. Zusammensetzung nach einem der vorhergehenden Ansprüche, **dadurch gekennzeichnet, dass** sie 0,01 bis 40 % verzweigten Sulfopolyester, vorzugsweise 0,1 bis 16 % verzweigten Sulfopolyester und besonders bevorzugt 0,5 bis 8 % verzweigten Sulfopolyester, bezogen auf das Gesamtgewicht der Zusammensetzung, enthält.

12. Zusammensetzung nach einem der vorhergehenden Ansprüche, **dadurch gekennzeichnet, dass** sie 0,01 bis 16 % Konditioniermittel und vorzugsweise 0,05 bis 4 % Konditioniermittel, bezogen auf das Gesamtgewicht der Zusammensetzung, enthält.

13. Zusammensetzung nach einem der vorhergehenden Ansprüche, **dadurch gekennzeichnet, dass** der oder die verzweigten Sulfopolyester in solubilisierter Form oder in Form einer Dispersion von festen Partikeln vorliegen.

14. Zusammensetzung nach einem der vorhergehenden Ansprüche, **dadurch gekennzeichnet, dass** das Konditioniermittel unlöslich ist und unter den Poly-α-Olefinen, fluorierten Ölen, pflanzlichen Ölen, natürlichen Wachsen, fluorierten Wachsen, fluorierten Gummis, Fettsäureestern, unlöslichen Siliconen, und Amidverbindungen, die mindestens eine Fettkette aufweisen, ausgewählt ist, wobei diese Stoffe auch in Form der Gemische vorliegen können.

15. Zusammensetzung nach Anspruch 1, **dadurch gekennzeichnet, dass** das kationische oder amphotere Polymer ausgewählt ist unter:
(a) kationischen Cellulosederivaten;
(b) Homopolymeren und Copolymeren von Dimethyldiallylammoniumhalogeniden;
(c) Homopolymeren und Copolymeren von Methacryloyloxyethyltrimethylammoniumhalogeniden;
(d) quartären Polyammoniumpolymeren;
(e) Copolymeren von Vinylpyrrolidon mit kationischen Einheiten; und
(f) kationischen Polysiloxanen.

16. Zusammensetzung nach Anspruch 15, **dadurch gekennzeichnet, dass** das kationische Polymer unter den quartären Celluloseesterderivaten, Copolymeren von Cellulose und einem wasserlöslichen quartären Ammoniummonomer, Cyclopolymeren, kationischen Polysacchariden, kationischen siliconhaltigen Polymeren, Vinylpyrrolidon/Dialkylaminoalkylacrylat oder Dialkylaminoalkylmethacrylat-Copolymeren, die gegebenenfalls quaternisiert sind, quartären Polymeren von Vinylpyrrolidon und Vinylimidazol, Polyamidoaminen und deren Gemischen ausgewählt ist.

17. Zusammensetzung nach einem der vorhergehenden Ansprüche, **dadurch gekennzeichnet, dass** die kationischen grenzflächenaktiven Stoffe in Wasser unlöslich sind und unter den Fettaminen und deren Salzen sowie den quartären Ammoniumsalzen ausgewählt sind.

18. Zusammensetzung nach Anspruch 17, **dadurch gekennzeichnet, dass** die Fettamine unter Dioctylamin, Stearyldimethylamin, Palmityldimethylamin, Oleocetyldimethylamin und Amidoaminen, wie Stearylamidoethyldiethylamin, Behenylamidopropyldimethylamin, Stearylamidopropyldimethylamin, Oleylamidopropyldimethylamin und Stearylamidoethyldimethylamin ausgewählt sind.

19. Zusammensetzung nach 17, **dadurch gekennzeichnet, dass** der kationische grenzflächenaktive Stoff vom Typ der quartären Ammoniumsalze unter den Verbindungen ausgewählt ist, die die folgende allgemeine Formel (I) aufweisen: 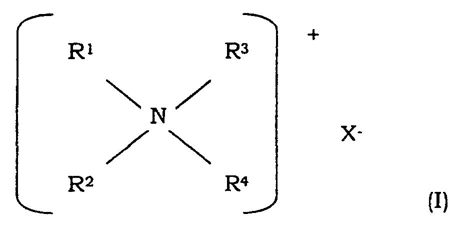 worin die Gruppen R₁ bis R₄, die gleich oder verschieden sind, eine aliphatische Gruppe mit 1 bis 22 Kohlenstoffatomen oder eine aromatische Gruppe, Alkoxy, Polyoxyalkylen, Alkylamid, Hydroxyalkyl, Aryl oder Alkylaryl mit 12 bis 22 Kohlenstoffatomen bedeuten; X ist ein Anion, das unter den Halogeniden, Phosphaten, Acetaten, Lactaten oder Alkylsulfaten ausgewählt ist;
oder den quartären Ammoniumsalzen von Imidazolinium der folgenden Formel (II): 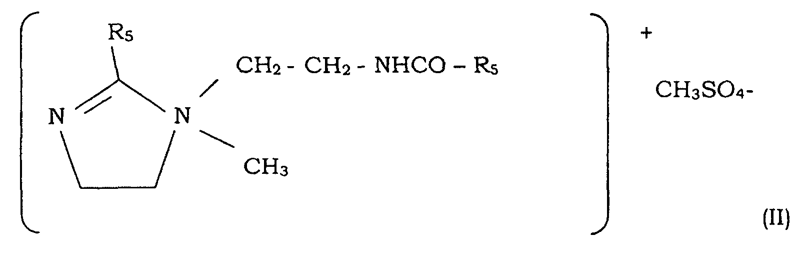 worin R₅ ein Gemisch von Alkenyl- und/oder Alkylgruppen mit 13 bis 21 Kohlenstoffatomen, die von Talgfettsäuren abgeleitet sind, bedeutet;
oder den quartären Diammoniumsalzen der Formel (III): 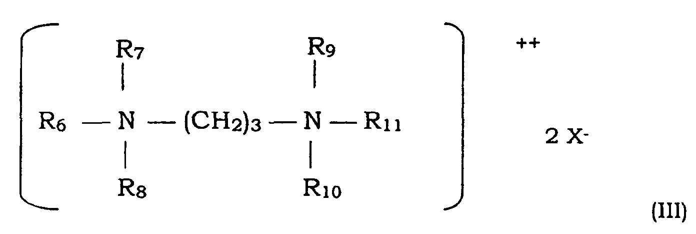 worin R₆ eine aliphatische Gruppe mit 16 bis 22 Kohlenstoffatomen bedeutet und die Gruppen R₇, R₈, R₉, R₁₀ und R₁₁ unter Wasserstoff oder einer Alkylgruppe mit 1 bis 4 Kohlenstoffatomen ausgewählt sind; X ist ein Anion, das unter den Halogeniden, Acetaten, Phosphaten und Sulfaten ausgewählt ist.

20. Zusammensetzung nach einem der vorhergehenden Ansprüche, **dadurch gekennzeichnet, dass** sie ferner übliche kosmetische Zusatzstoffe enthält, beispielsweise Weichmacher und Neutralisationsmittel.

21. Zusammensetzung nach einem der vorhergehenden Ansprüche, **dadurch gekennzeichnet, dass** sie in Form einer versprühbaren Zusammensetzung, als Schaum, Gel oder Lotion vorliegt.

22. Zusammensetzung nach einem der vorhergehenden Ansprüche, **dadurch gekennzeichnet, dass** sie ein Lösungsmittel enthält, das unter Wasser, Alkoholen oder wässrig-alkoholischen Gemischen ausgewählt ist.

23. Zusammensetzung nach einem der vorhergehenden Ansprüche, **dadurch gekennzeichnet, dass** sie ferner ein Treibmittel in einer geeigneten Menge enthält.

24. Zusammensetzung nach Anspruch 23, **dadurch gekennzeichnet, dass** das Treibmittel aus üblichen komprimierten oder verflüssigen Gasen besteht, vorzugsweise komprimierter Luft, komprimiertem Kohlendioxid oder komprimiertem Stickstoff oder aus einem in der Zusammensetzung löslichen oder unlöslichen Gas, wie Dimethylether, fluorierten Kohlenwasserstoffen oder nicht fluorierten Kohlenwasserstoffen und deren Gemischen.

25. Aerosolvorrichtung, die aus einem Behälter, der eine Aerosolzusammensetzung enthält, die aus einer flüssigen Phase (oder Flüssigkeit), die eine Zusammensetzung nach einem der Ansprüche 1 bis 20 in einem geeigneten Lösungsmittel enthält, und einem Treibmittel besteht, sowie einer Einrichtung zur Verteilung dieser Aerosolzusammensetzung besteht.

26. Verfahren zur Behandlung von Keratinfasern und insbesondere zur Behandlung der Haare, **dadurch gekennzeichnet, dass** auf die Fasern die Zusammensetzung nach einem der Ansprüche 1 bis 24 aufgebracht wird, bevor die Haare in Form gebracht werden oder nachdem die Frisur geformt wurde.

27. Verwendung einer Zusammensetzung nach einem der vorhergehenden Ansprüche 1 bis 24 in einer kosmetischen Formulierung zur Frisurengestaltung oder zur Herstellung einer solchen Formulierung.
